# EUROPEAN PATENT APPLICATION

(11) **EP 3 424 922 A1**
(43) Date of publication of application: **09.01.2019**
(21) Application number: 17180401.6
(22) Date of filing: 07.07.2017
(51) Int. Cl.: C07D 405/12, G01N 33/68

(54) **PHOTO-SWITCHABLE CHEMICAL INDUCERS OF DIMERIZATION FOR CONTROL OF PROTEIN FUNCTION IN CELLS BY LIGHT**

(71) Applicant: Max-Planck-Gesellschaft zur Förderung der Wissenschaften e.V., 80539 München (DE)
(72) Inventor: WU, Yaowen, 44265 Dortmund (DE); CHEN, Xi, 44149 Dortmund (DE)
(74) Representative: Arth, Hans-Lothar

(57) **Abstract**

The present application refers to photo-switchable chemical inducers of dimerization for control of protein interactions in cells by light. A compound, a test system, methods and uses are disclosed how the invention can be applied in the investigation of intracellular protein interactions. The system is composed of a compound of the general formula (I) as the photo-caged dimerizer, with the ability to covalently bind to HaloTag and a high affinity binding to eDHFR, respectively. The system can be activated and deactivated selectively on illumination with light under different irradiation conditions.

## Description

The present application refers to photo-switchable chemical inducers of reversible dimerization for control of protein function in cells by light.

Chemical inducers of dimerization (CIDs or "dimerizers") have shown to be a powerful tool for modulating protein interactions. They have been widely used in a variety of biological studies in particular in the field of signal transduction.

Methods to perturb and control the intracellular activity and/or localizations of proteins are enormously useful to test a variety of biological processes and cellular networks. A dimerizer binds simultaneously to two protein modules to form a homodimer or heterodimer. Dimerizers allow for the control of protein-protein interactions and have been shown to be a powerful tool for the investigation of biological events. In these applications, proteins of interest are fused to the binding modules recognized by the respective dimerizer. Treatment with the dimerizer brings fusion proteins together. In this way protein function and thus the downstream cellular events can be modulated in real time.

Rapamycin was the first naturally occurring dimerizer described in literature. Up to now this compound has been extensively analyzed. Rapamycin mediates the heterodimerization of FK506-binding protein (FKBP) and the FKBP-rapamycin-binding (FRB) domain of kinase mTOR (mammalian target of rapamycin) protein. Rapamycin has been broadly used for controlling protein function in many biological processes, including gene transcription, signal transduction, post-translational protein modification and protein degradation.

However, all known CIDs suffer from a long period of time, up to several minutes, required to cross the plasma membrane before action and that it distributes to the entire cell. Therefore, traditional CIDs lack spatial and temporal resolution and therefore rendering it difficult to study acute and local cellular process without affecting other regions of the cell.

Photosensitive proteins, e.g. LOV domain, phytochromes, photochromic fluorescent protein, and cytochromes enable control of protein interactions by light. They have been applied to optogenetic control of protein-protein interactions, cell signaling, cell motility, and ion channel functions. More specifically, the dimerization of two photosensitive protein domains is triggered by illumination at a certain wavelength, and is reversed in the dark state or by illumination at another wavelength. However, optogenetic tools using photosensitive proteins usually require constant illumination. Continuous illumination, in particular in the blue light region, may be harmful to cells. In addition, photosensitive proteins display broad absorption spectra (PhyB: 550-800nm; LOV1: 400-500nm; Cry2: 390-500nm), which overlap with commonly used fluorescent proteins that are used to read out dimerization process and cellular responses. Furthermore, photosensitive proteins typically display weak bindings and therefore they are not well suited to study most of the protein dimerization processes that require a stronger binding affinity.

Thus, there remains a high demand for photo-switchable dimerization systems for controlling protein functions with minimal cellular interference by light. An ideal photo-switchable CID system would allow for reversibly controlling protein function with minimal intracellular interactions by light.

Therefore it is the task of the present invention to provide a dimerizer system that allows for forming homodimers or heterodimers of any intracellular peptidic compound and/or similar exogenous compound brought into a cell, preferably a eukaryotic cell, which enables testing their respective intracellular interactions without activating or biasing other intracellular signal pathways. Ideally, the inventive dimerizer should work as a bioorthogonal system.

Surprisingly, this problem can be solved by the compounds, uses and methods disclosed in the independent claims of the present application. Further advantageous embodiments and applications can be found in the dependent claims, the description and the figures.

The inventive dimerizers are compounds of general formula (I)

Hal-(CH₂)₆-**F¹-P-F²-E** (**I**)

wherein
Hal is selected from -Cl, -Br and -I;
**E** is selected from: 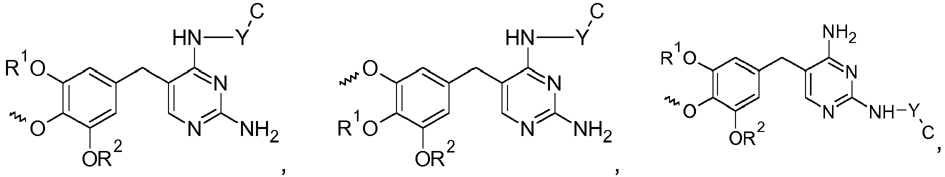 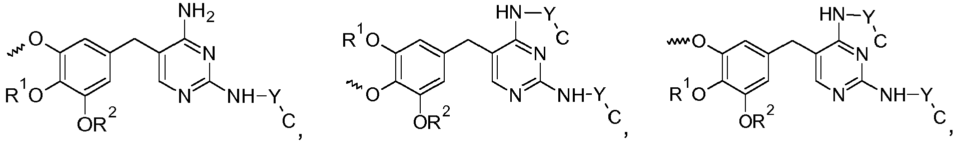 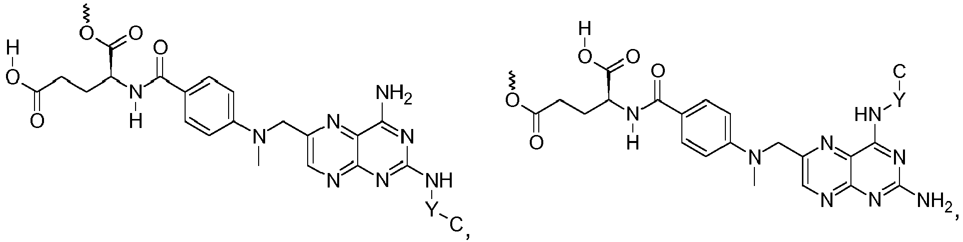 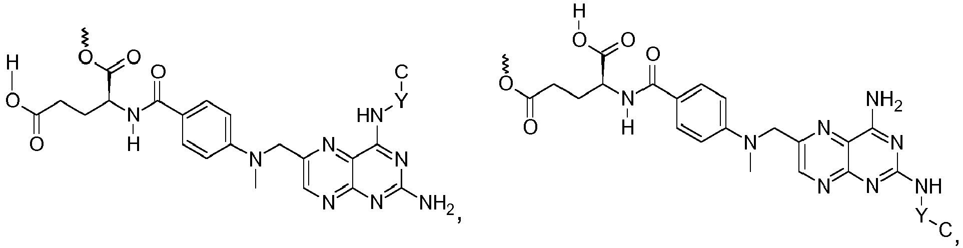 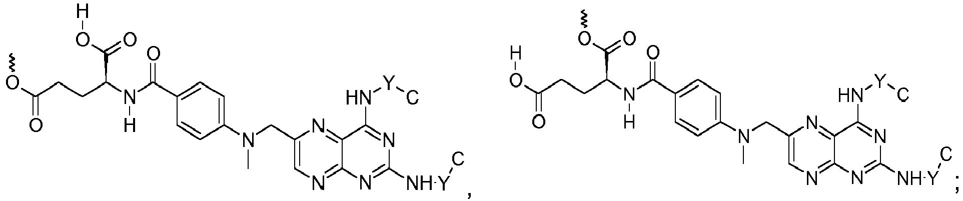 wherein R¹ and R² are independently of each other selected from: -H, -CH₃, -C₂H₅, -C₃H₇, -Ph, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, cyclo-C₃H₅, cyclo-C₄H₇, cyclo-C₅H₉, cyclo-C₆H₁₁;
Y represents a bond, -CH₂-, -NHR³⁴-, -O-, -S-, -C(=O)-O-, -O-C(=O)-, -CO-, -NHC(=O)-, -C(=O)NH-, -NR³⁴-C(=O)-, -C(=O)NR³⁴-, -NH-C(=S)-, or -C(=S)NH-;
C is selected from -C1, -C2, -C3, -C4, -C5, -C6, -C7, -C8, -C9, or -C10;

| | | | |
|---|---|---|---|
| C1 is | 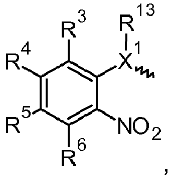 | C2 is | 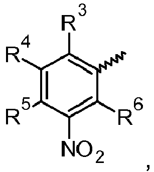 |
| C3 is | 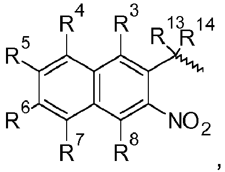 | C4 is | 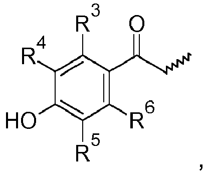 |
| C5 is | 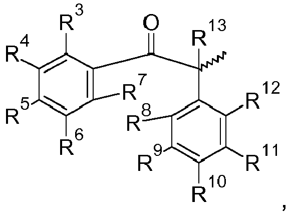 | C6 is | 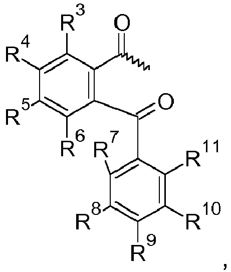 |
| C7 is | 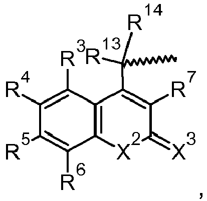 | C8 is | 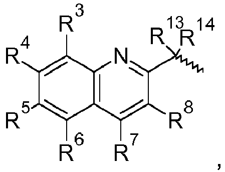 |
| C9 is | 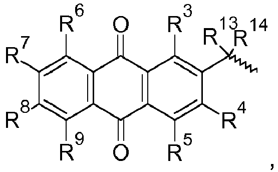 | C10 is | 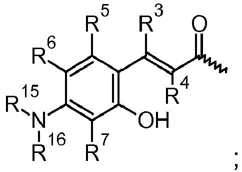 |

P is selected from -P1-, -P2-, -P3-, -P4-, -P5-, -P6-, -P7-, -P8-, -P9-, -P10-, -P11-, and -P12-; wherein

| | | | |
|---|---|---|---|
| P1 is | 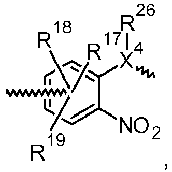 | P2 is | 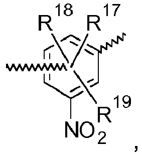 |
| P3 is | 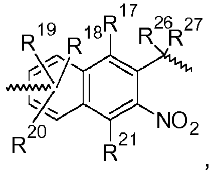 | P4 is | 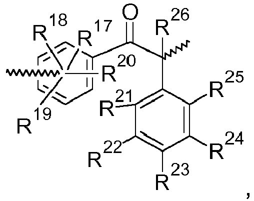 |
| P5 is | 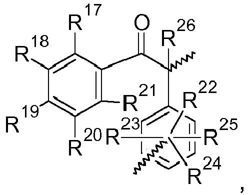 | P6 is | 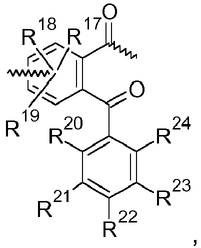 |
| P7 is | 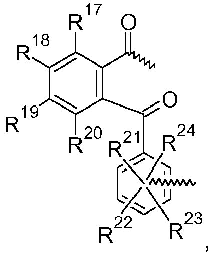 | P8 is | 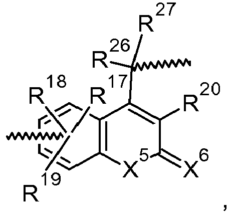 |
| P9 is | 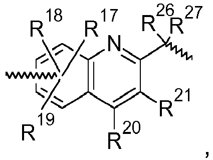 | P10 is | 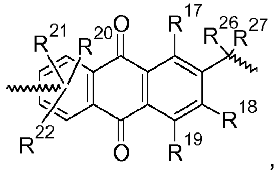 |
| P11 is | 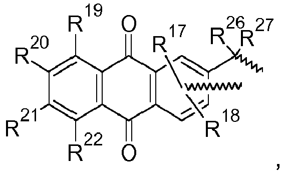 | P12 is | 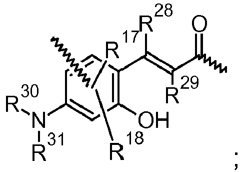 |

wherein if C is C1, P cannot be P1 or P2;
wherein if C is C2, P cannot be P1 or P2;
wherein if C is C3, P cannot be P3;
wherein if C is C4, P cannot be P4 or P5;
wherein if C is C5, P cannot be P4 or P5;
wherein if C is C6, P cannot be P6 or P7;
wherein if C is C7, P cannot be P8;
wherein if C is C8, P cannot be P9;
wherein if C is C9, P cannot be P10 or P11;
wherein if C is C10, P cannot be P12;
X¹ is either 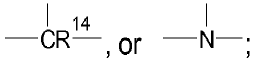 X⁴ is either 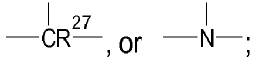 X² and X⁵ are independently of each other selected from: -O-, -S-, -NH-, and -NR³²-;
X³ and X⁶ are independently of each other selected from: =O, =S, =NH, and =NR³³;
F¹ is -A¹-L^{A}-B¹- and F² is -A²-L^{B}-B²-, wherein
A¹, A², B¹ and B² represent independently of each other -CH₂-, -NH-, -O-, -S-, -CO-, -NH-CO-, -CO-NH-, -NH-CO-NH-, -O-CO-, -O-CO-O-, -NH-CO-O-, -O-CO-NH-, -NH-CO-CH₂-, -CH₂-CO-NH- and -CO-O-; L^{A} and L^{B} represent independently of each other -(CH₂)ₘ₁-, -(CH₂)ₘ₂-, -(CH₂)ₘ₁-CHR³⁵-(CH₂)ₘ₂-, -(CH₂)ₘ₁-CR³⁶R³⁷-(CH₂)ₘ₂-, -(C₂H₄O)ₘ₁-, -(C₂H₄O)ₘ₂-, -(OC₂H₄)ₘ₁-, -(OC₂H₄)ₘ₂-, -(CH₂)ₘ₅-(C₂H₄O)ₘ₆-, -(CH₂)ₘ₅-(OC₂H₄)ₘ₆-, -(C₂H₄O)ₘ₅-(CH₂)ₘ₆-, -(OC₂H₄)ₘ₅-(CH₂)ₘ₆-, -(CH₂)ₘ₇-(C₂H₄O)ₘ₈-, -(CH₂)ₘ₇-(OC₂H₄)ₘ₈-, -(C₂H₄O)ₘ₇-(CH₂)ₘ₈-, -(OC₂H₄)ₘ₇-(CH₂)ₘ₈-, -(CH₂)ₘ₁-(C₂H₄O)ₘ₂-(CH₂)ₘ₅-, -(CH₂)ₘ₁-(OC₂H₄)ₘ₂-(CH₂)ₘ₅-, -(CH₂)ₘ₆-(C₂H₄O)ₘ₇-(CH₂)ₘ₈-, -(CH₂)ₘ₆-₍OC₂H₄)ₘ₇-(CH₂₎ₘ₈₋, -O-C₆H₄-, -m-C₆H₄-, -p-C₆H₄-, 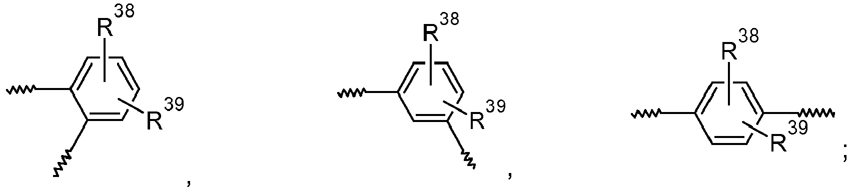 R³ to R¹⁴, R¹⁷ to R²⁹ and R³⁵ to R³⁹ represent independently of each other -H, -F, -Cl, -Br, -I, -CF₃, -NH₂, -N(CH₃)₂, -N(C₂H₅)₂, 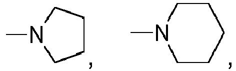 -OH, -OCH₃, -OC₂H₅, -OC₃H₇, -OCH₂COOH, -N(CH₂COOH)₂, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH₂NH₂, -CH₂OH, -CH₂SH, -CH₂-CH₂NH₂, -CH₂-CH₂SH, -C₆H₄-OCH₃, -C₆H₄-OH, -CH₂-CH₂-OCH₃, -CH₂-CH₂OH, -CH₂-OCH₃, -CH₂-C₆H₄-OCH₃, -CH₂-C₆H₄-OH, or
two neighbouring residues R³ to R¹² and R¹⁷ to R²⁵ form a benzo ring, or three neighbouring residues R³ to R¹² and R¹⁷ to R²⁵ form a 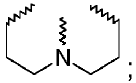 R¹⁵, R¹⁶, R³⁰ to R³⁴ represent independently of each other -H, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH₂NH₂, -CH₂OH, -CH₂SH, -CH₂-CH₂NH₂, -CH₂-CH₂SH, -C₆H₄-OCH₃, -C₆H₄-OH, -CH₂-CH₂-OCH₃, -CH₂-CH₂OH, -CH₂-OCH₃, -CH₂-C₆H₄-OCH₃, -CH₂-C₆H₄-OH,
m1, m2, m5, m6, m7 and m8 represent independently of each other an integer from 1 to 20; m3 and m4 represent independently of each other an integer from 0 to 5.

Herein, **E** is an *Escherichia coli* dihydrofolate reductase (eDHFR) ligand caged by one or two photo-cleavable groups Y-C; **P** is a photo-cleavable linker and **F¹** and **F²** are linker defined as above or preferably selected from the group comprising or consisting of polyethylene glycol (PEG), triethylene glycol, tetraethylene glycol (TEG), alkyl chain with up to 30 carbon atoms, phosphodiesters, glycosides, amides, esters, diesters, thioesters, aldol products, acetate moieties, polyethylenes, isoprenoids, phenyl groups, aromatic groups, hetrocyclic groups, ethers, thioethers, and imines.

Preferred are also the intermediate compounds of the general formula I-1,

Hal-(CH₂)₆-**F¹-P-F²-E** (**I-1**)

wherein
the moieties **Hal, F¹**, **P, F²**, **and E** have the meanings as defined above or as defined in claim 1 and wherein the residue -YC present in the moiety **E** represents hydrogen (-H).

As described above, the inventive compound comprises a halohexane [-(CH₂)₆-Hal] moiety which is a ligand that binds to HaloTag labeled proteins.

The photo-cleavable linker **P** can be cleaved upon irradiation with light and the ligand **E** is modified with a photo-caging group C to block its binding with eDHFR. The photo-caging group C is chosen to be removed under different irradiation conditions than the photo-cleavable linker **P** is cleaved which enables activating the ligand **E** of the photo-switchable CID first, without affecting the photo-cleavable linker **P** and subsequently deactivating the photo-switchable CID under irradiation conditions under which the linker **P** is cleaved. Consequently, by removing the photo-caging group C without cleavage of the linker **P** the dimerization of two proteins is induced where one has a HaloTag binding domain and another binds an eDHFR ligand. The dimerization can be reversed by cleavage of the linker **P** upon irradiation with light.

In that regard the chemical inducers of dimerization of the present invention are **photo-switchable** which means that under distinct irradiation conditions the dimerization can be induced and reversed with light independently. Herein, the chemical inducers of dimerization of the present invention are also denoted as CIDs, psCID, or dimerizers. However, dimerization and reversal of dimerization (dedimerization) cannot be induced under the same irradiation conditions. In other words, irradiation conditions for dimerization and dedimerization are orthogonal, i.e. while the dimerization is induced by irradiation with light under irradiation condition A the dimerization of the two proteins via the chemical inducers of dimerization of the present invention is reversed by irradiation with light under irradiation condition B. Irradiation condition A and B may differ in the used wavelength and/or intensity of light.

The term **"photoactivation"** refers herein to the removal the photo-caging group C of the photo-switchable CID of the present invention with light under irradiation condition A. Upon decaging of the ligand **E**, a second protein can now bind to the eDHFR ligand **E.**

The term **"photodeactivation"** refers herein to the cleavage of the linker **P** of the photo-switchable CID of the present invention with light under irradiation condition B.

Herein, HaloTag refers to a modified haloalkane dehalogenase. HaloTag ligands, such as chlorohexane derivatives, can bind with HaloTag in a rapid and covalent fashion. On the other hand, eDHFR refers to Escherichia coli dihydrofolate reductase. eDHFR ligands, such as TMP (5-(3,4,5-trimethoxybenzyl)pyrimidine-2,4-diamine, trimethoprim), confer non-covalent binding with eDHFR with high affinity (nM range) and specificity. TMP ligand could also be replaced by other eDHFR binding ligands, such as methotrexate (MTX).

TMP has the following structure:

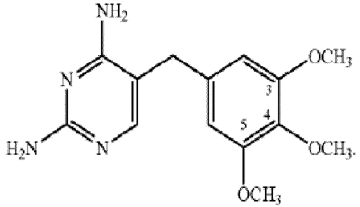

MTX has the following structure:

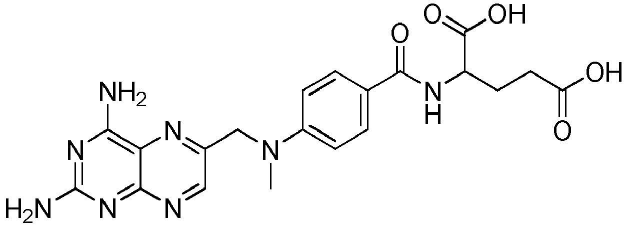

More specifically, in one embodiment this photo-switchable CID has either one of the two following structures (**II-A**) or (**II-B**), wherein the halohexane is a chlorohexane derivative for binding with HaloTag and the *Escherichia coli* dihydrofolate reductase (eDHFR) ligand E is trimethoprim (TMP) for binding with eDHFR. Either one or both of the two primary amino groups on the pyrimidine in the TMP moiety are modified with a photo-caging group C via a linker Y, 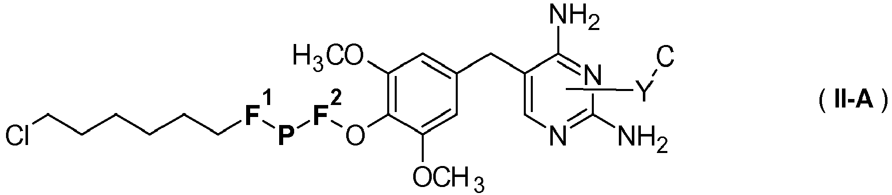 or 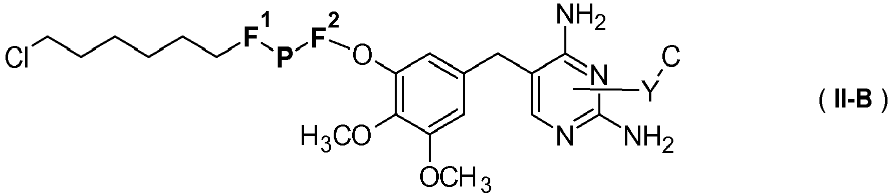 wherein **F¹**, **P**, **F²**, Y and C have the meanings as defined above.

In a preferred embodiment of the present invention the photo-cleavable linker **P** is an ortho-nitrobenzyl group and ligand **E** is TMP caged by a coumarinyl group. Thus, in one embodiment of the present invention the photo-switchable chemical inducer of dimerization has the following general formula. 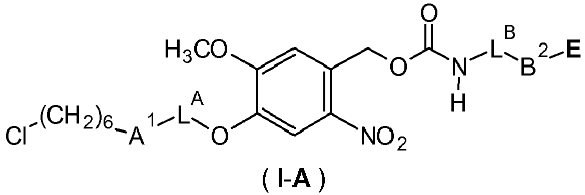 wherein
**E** is selected from: 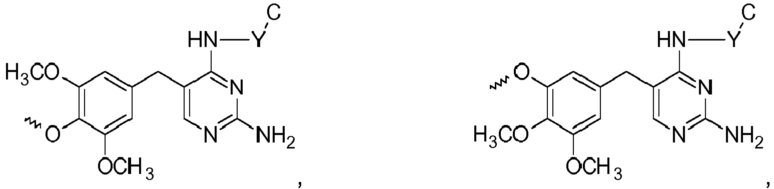 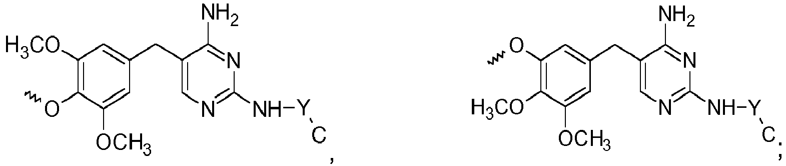 Y-C is 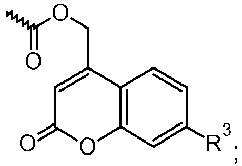 R³ is selected from -NH₂, -N(CH₃)₂, -N(C₂H₅)₂, 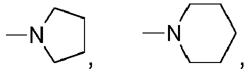 and -N(CH₂COOH)₂; and
A¹, L^{A}, L^{B} and B² have the meanings as defined above.

Preferred are also the intermediate compounds of the general formula I-1-A, 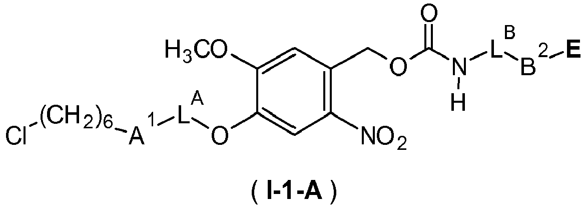 wherein
the moieties **A¹, B², L^{A}, L^{B}**, **and E** have the meanings as defined above or as defined in claim 2 and wherein the residue -YC present in the moiety **E** represents hydrogen (-H).

In an alternative embodiment of the present invention the photo-switchable chemical inducer of dimerization has the general formula (**I-B**) 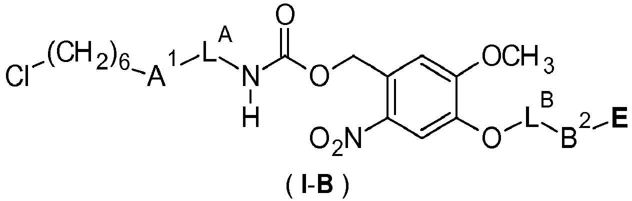 wherein
E is selected from: 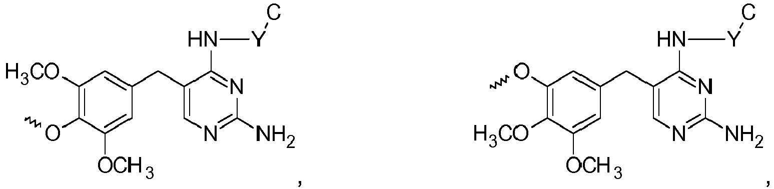 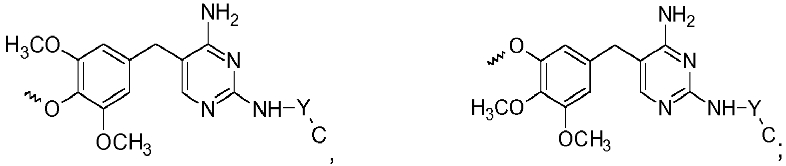 Y-C is 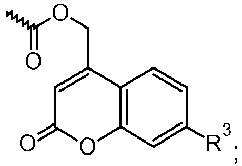 R³ is selected from -NH₂, -N(CH₃)₂, -N(C₂H₅)₂, 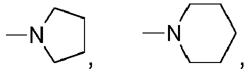 and -N(CH₂COOH)₂; and
wherein A¹, L^{A}, L^{B} and B² have the meanings as defined above.

Preferred are also the intermediate compounds of the general formula I-1-B, 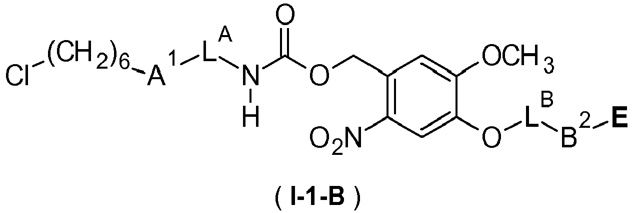 wherein
the moieties **A¹, B², L^{A}**, **L^{B}**, **and E** have the meanings as defined above or as defined in claim 3 and wherein the residue -YC present in the moiety **E** represents hydrogen (-H).

Especially preferred is the intermediate compound 5

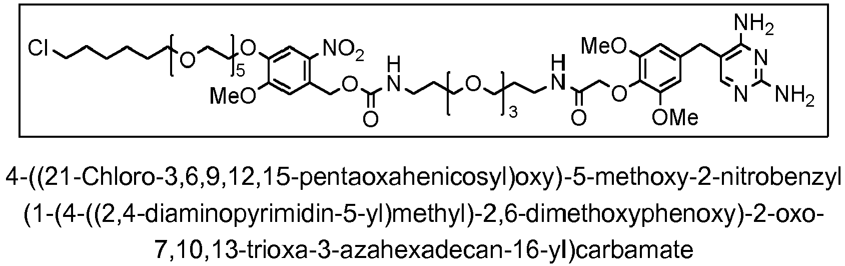

Particularly preferred are the following photo-switchable chemical inducers of dimerization: 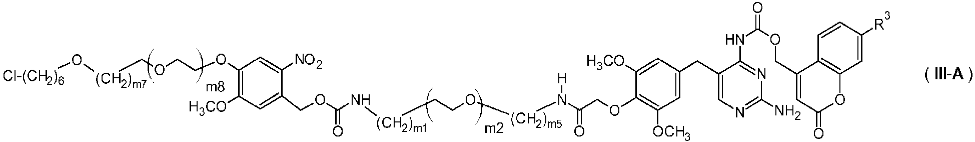 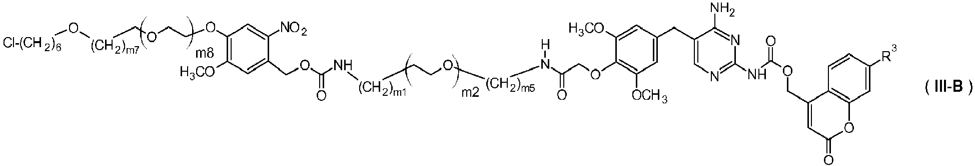 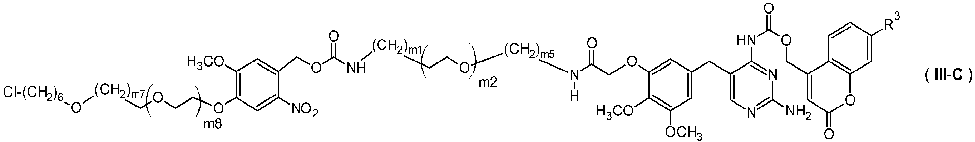 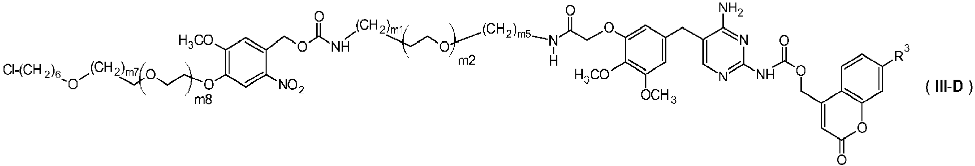 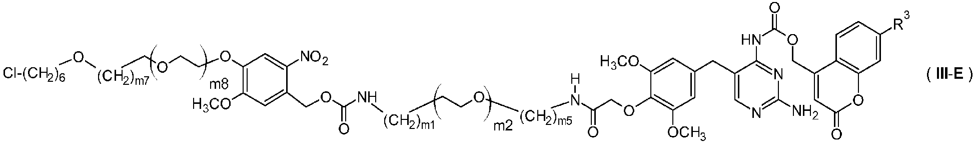 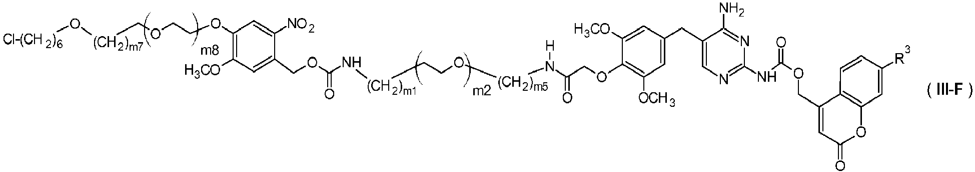 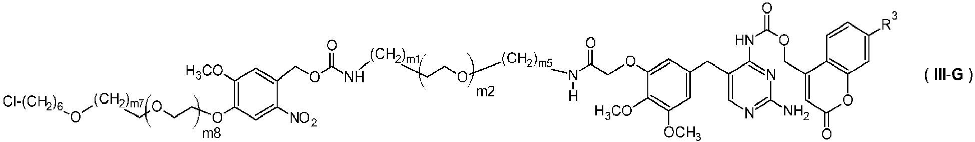 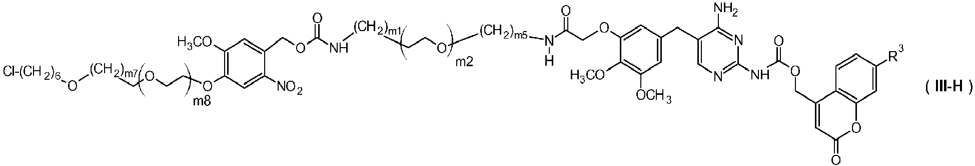 wherein R³ is selected from -NH₂, -N(CH₃)₂, -N(C₂H₅)₂, 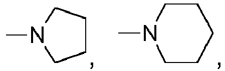 and -N(CH₂COOH)₂; and m1, m2, m5, m7 and m8 represent independently from each other an integer from 1 to 20.

In a particularly preferred embodiment of the present invention, the chemical inducer of dimerization is 4-CmTMP-PC-Cl having the following structure:

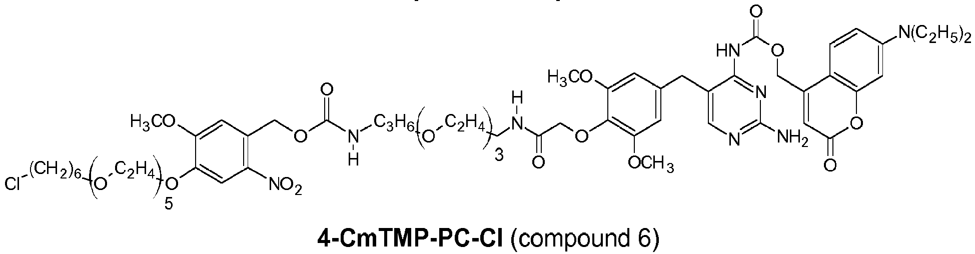

4-CmTMP-PC-Cl features a chlorohexyl group for covalent binding to HaloTag, a diethylaminocoumarinyl caged TMP, and a 2-nitrobenzyl photocleavable moiety. A detailed description of the synthesis of 4-CmTMP-PC-Cl (compound 6) is given in Example 1.

While it is not necessary as shown in the structure above that the linker is connected to the oxygen atom at C₄ position in 4-CmTMP-PC-Cl, it is also possible to connect the linker to the oxygen at position C₃ or at position C₅ of the dimethoxyphenyl moiety. That means that the linker can be bound to the oxygen atom of one of the two methoxy groups on C₃ and C₅. Binding of the linker to the oxygen atom at C₃ or C₅ does not abolish the activity of the obtained molecule.

Fusion proteins or chimeric proteins are proteins created through the joining of two or more DNA-sequences, mostly created through genetic engineering, which originally coded for separate proteins or protein domains. The fusion proteins according to the invention may be a recombinant fusion protein created through genetic engineering of a fusion gene. This typically involves removing the stop codon from a cDNA sequence coding for the first protein, then appending the cDNA sequence of the second protein in frame followed by a stop codon. The resulting DNA sequence will then be expressed by a cell as a single protein. The fusion protein may be engineered to include the full sequence of both original proteins (HaloTag or bacterial DHFR and protein of interest) or only a portion of either. Fusion proteins of at least the HaloTag and a bacterial DHFR, in particular eDHFR, respectively, with the two proteins the interactions of which shall be tested can be generated according to conventional methods. Thus, preferred fusion proteins according to the invention may comprise the binding domain of HaloTag or respectively the binding domain of a bacterial DHFR, in particular eDHFR, and a protein or protein domain of interest (POI, for example Bicaudal D2 (1-594) and Rab5). Furthermore the fusion proteins may also comprise some additional amino acids for example as a linking moiety or spacer between two proteins or protein domains. Additional amino acids may also be caused by strategy of genetic engineering. The fusion protein preferably comprise in addition a component for identification and/or purification of the fusion proteins, like a GST protein, FLAG peptide, a Myc-Peptide, a human influenza hemagglutinin (HA)-peptide, a hexa-his peptide (6xHis-tag), a fluorescent protein (variants of GFP, CFP, YFP, BFP, RFP, far-red FP), or a protein tag that can be labeled by a fluorescent dye (e.g. SNAP-tag, etc). The DNA constructs coding for the fusion proteins are going to be transfected to the test cell culture by conventional methods.

In another preferred embodiment these DNA constructs are going to be expressed under the control of a suitable promoter gene sequence. For example, the cytomegalovirus (CMV) promoter is one of the most commonly used promoters for expression of transgenes in mammalian cells. Ideally, the promoter can be easily regulated by the experimenter. Suitable promoter systems include for example tetracycline controlled promoter systems. It is therefore preferred if the DNA constructs include inducible promoter systems that are regulated by particular chemical or physical factors.

Thus the present invention also refers to a dimerization system for testing intracellular protein interaction in eukaryotic cells, comprising
a) a compound of the general formula (I);
b) fusion protein 1 generated from a test compound (plasmid) 1 and at least HaloTag; and
c) fusion protein 2 generated from a test compound (plasmid) 2 and at least the TMP binding domain of a bacterial DHFR.

Further, the present invention refers also to a dimerization system with the above-mentioned components a) - c), wherein test compound 1 or test compound 2 expresses a fluorescent protein.

Suitable fluorescent proteins can be selected from various variants of blue fluorescent protein (e.g., BFP, TagBFP, etc), cyan fluorescent proteins (e.g., mTurquoise2, ECFP, etc.), yellow fluorescent proteins (e.g., YFP, Citrine), green fluorescent proteins (e.g., EGFP), red fluorescence protein (e.g., mCherry), and far-red fluorescent proteins (e.g, mCardinal).

Further, the present invention refers also to a dimerization system with the above-mentioned components a) - c), wherein test compound 1 or test compound 2 expresses a protein tag that can be specifically labeled by a fluorescent dye. Suitable protein tags include SNAP-tag, CLIP-tag, His-tag, tetracysteine-tag, D4-tag, HyRe-tag, SpyTag, E3-tag, FKBP(F37V)-tag, PYP-tag, BL-tag, Cutinase-tag, ACP-tag, AP-tag, Q-tag, LAP-tag, formylglycine generating enzyme substrate-tag, Sortase A substrate-tag, CAAX-tag, Split intein-tag, AnkX substrate-tag, Tub-tag, unnatural amino acids (UAAs), and others.

Here, we term the dimerization system for testing intracellular protein interaction in eukaryotic cells according to the invention a chemo-optogenetic system since the system combines engineered fusion proteins with their stimulation or proteins by light by the inventive dimerizer.

According to the invention a method for testing intracellular protein interaction in eukaryotic cells is disclosed, comprising the following steps:
a) Providing, transfecting and expressing the DNA sequence of a fusion protein 1 comprising a test compound 1 and at least HaloTag;
b) providing, transfecting and expressing the DNA sequence of a fusion protein 2 comprising a test compound 2 and at least the TMP binding domain of a bacterial DHFR;
c) adding a compound of general formula (I) to cells, preferably eukaryotic cells, and letting it pass the plasma membrane;
d) activating the compound of general formula (I) with light under irradiation condition A;
e) measuring the physiological effect by determining the change in a selected test parameter system;
f) deactivating the compound of general formula (I) with light under irradiation condition B.

In an alternative embodiment, the method for testing intracellular protein interaction in eukaryotic cells comprises the following steps
a) Providing, transfecting and expressing the DNA sequence of a fusion protein 1 comprising a test compound 1 and at least HaloTag;
b) providing, transfecting and expressing the DNA sequence of a fusion protein 2 comprising a test compound 2 and at least the TMP binding domain of a bacterial DHFR;
c) adding a compound of general formula (I) to cells, preferably eukaryotic cells, and letting it pass the plasma membrane;
d) controlling the physiological effect by photoactivation and photodeactivation of the dimerization process using either different wavelengths of light or different dose of light illumination; and
e) determining the change in a selected test parameter system.

In a further embodiment, the method for testing intracellular protein interaction in eukaryotic cells comprises the following steps
a) Providing, transfecting and expressing the DNA sequence of a fusion protein 1 comprising a test compound 1 and at least HaloTag;
b) providing, transfecting and expressing the DNA sequence of a fusion protein 2 comprising a test compound 2 and at least the TMP binding domain of a bacterial DHFR;
c) adding compound of the general formula (I) to cells and letting them pass the plasma membrane;
d) activating and/or deactivating the compound of general formula (I) with light under irradiation condition A for activation and under irradiation condition B for deactivation; and
e) determining the change in a selected test parameter system upon activation and/or deactivation of the compound of general formula (I).

The method for testing intracellular protein interaction in eukaryotic cells may further comprise the step c') between step c) and step d):
c') after incubation, briefly wash the cell to remove the unreacted compound.

As said before, the interaction introduced by a compound according to formula (I) after activation with light under irradiation condition A is reversible by deactivation under irradiation condition B. Therefore the present invention refers also to above said method, wherein the generated effect is reverted by irradiating the cells, preferably eukaryotic cells, with light under irradiation condition B which causes the photo-cleavable linker P of the compound according to formula (I) to break.

In this method test compound 1 and test compound 2 are selected independently from each other among proteins, protein domains, peptides, polypeptides, glycopeptides, proteins with secondarily modified amino acids, peptides or proteins with protecting groups, saccharides, small molecules, lipids, oligonucleic acids like DNA or RNA.

In a particularly preferred embodiment the bacterial DHFR in this method is eDHFR. Thus Method for testing intracellular protein interaction in cells, comprises the following steps:
a) Providing, transfecting and expressing the DNA sequence of a fusion protein 1 comprising a test compound 1 and at least HaloTag;
b) providing, transfecting and expressing the DNA sequence of a fusion protein 2 comprising a test compound 2 and at least the TMP binding domain of eDHFR;
c) adding compound according to claim 1 to cells and letting them pass the plasma membrane;
d) activating and/or deactivating the compound of general formula (I) with light under irradiation condition A for activation and under irradiation condition B for deactivation; and
e) determining the change in a selected test parameter system.

In this method activation of the chemical inducer of dimerization of general formula (I) is (photo-)activated by irradiation with light under irradiation condition A and (photo-)deactivated by irradiation with light under irradiation condition B. Preferably, the irradiation condition A corresponds to irradiation with an Argon laser at a wavelength of 458 nm. It is also preferred that the irradiation condition B corresponds to irradiation with a laser diode at a wavelength of 405 nm.

In a further embodiment the irradiation condition A for photoactivation of a chemical inducer of dimerization of general formula (I) the irradiation condition B for photodeactivation corresponds to irradiation with a laser at the same wavelength, wherein the applied fluence of the laser under the irradiation condition A is lower than under the irradiation condition B. The term "fluence" herein refers to the optical energy per unit area delivered by a laser pulse. Particularly preferred is when the fluence of the laser under the irradiation condition A is 1/2, more preferred 1/4, even more preferred 1/8, even more preferred 1/16, even more preferred one 1/32 and at most preferred 1/64 of the fluence of the laser under the irradiation condition B.

In a further embodiment the irradiation condition A for photoactivation of a chemical inducer of dimerization of general formula (I) corresponds to irradiation with a laser diode at a wavelength of 405 nm and the irradiation condition B for photodeactivation of a chemical inducer of dimerization of general formula (I) corresponds to irradiation with a laser diode at a wavelength of 405 nm, wherein the applied fluence of the laser under the irradiation condition A is lower than about 0.99 J/cm² and the applied fluence of the laser under the irradiation condition B is higher than about 0.99 J/cm². Particularly preferred is when the applied fluence of the laser under the irradiation condition A is in the range of 0.06 J/cm² to 0.99 J/cm², more preferably in the range of 0.12J/cm² to 0.99 J/cm², even more preferably in the range of 0.25 J/cm² to 0.99 J/cm² and most preferably in the range of 0.45 J/cm² to 0.99 J/cm². It is also preferred when the applied fluence of the laser under the irradiation condition B is in the range of 2 J/cm² to 32 J/cm², more preferably in the range of 4 J/cm² to 32 J/cm², even more preferably in the range of 8 J/cm² to 32 J/cm² and most preferably in the range of 16 J/cm² to 32J/cm².

The present application is also directed to this method, wherein test compound 1 and test compound 2 is a physiological peptidic compound, respectively.

Suitable test parameter systems may be selected depending on the test compounds. Test parameter systems may be selected from the group comprising or consisting of: measurement of an enzymatic reaction like phosphorylation or dephosphorylation, proteolysis, ubiquitination or deubiquitination, glycosylation, acetylation or deacetylation, determining the transcription or translation of a gene of interest or a reporter gene, determining the activation of a signaling pathway, microscopic measurement of protein or peptide localization in cells, dimerization (binding) assay by fluorescent resonance energy transfer (FRET), bioluminescence resonance energy transfer (BRET), protein compliment assay (PCA), fluorescence anisotropy, fluorescence correlation spectroscopy (FCS) / fluorescence cross-correlation spectroscopy (FCCS), yeast two-hybrid (Y2H), size exclusion chromatography, co-sedimentation, pull-down, microarray, isothermal titration calorimetry (ITC), surface plasmon resonance (SPR), microscale thermophoresis, and chemical field-effect transistors. The transcription of a transporter gene may be determined by measurement of the corresponding mRNA or of the protein the gene encodes for. Alternatively, the result of an enzymatic reaction may be determined if the gene of interest or the reporter gene encodes for an enzyme.

A likewise interesting application is to test the interactions between a pharmaceutical drug (a pharmaceutical drug authorized for medical use in humans and/or animals, a corresponding prodrug or a drug candidate under investigation) and a physiological peptidic compound. Thus the inventive method can be used in an arrangement, wherein test compound 1 is a pharmaceutical drug and test compound 2 is a physiological peptidic compound, or test compound 1 is a physiological peptidic compound and test compound 2 is a pharmaceutical drug.

This test arrangement can be varied by investigating a gene regulating protein and a pharmaceutical drug. Thus the inventive method can be carried out in such a way that test compound 1 is a gene regulating protein and test compound 2 is a pharmaceutical drug, or test compound 1 is a pharmaceutical drug and test compound 2 is a gene regulating protein.

In molecular biology the use of commercially available kits is becoming more popular. They include all components needed for performing a specific test and/or analysis arrangement in reasonable amounts. Often some or all of the components are already dissolved in a suitable test solution or buffer in useful concentrations.

Therefore this invention also applies to a kit, comprising
a) a compound of the general formula (I);
b) the nucleotide sequences or the vectors including the nucleotide sequences coding for at least the binding domains of HaloTag and a bacterial DHFR.

Optionally, the kit according to the invention can also comprise
c) chemical means for promoting transfection.

These means depend on the transfection method to be used. Possible chemical transfection methods include calcium phosphate method, dendrimer method, lipofection, and polycation-based methods.

### Figures

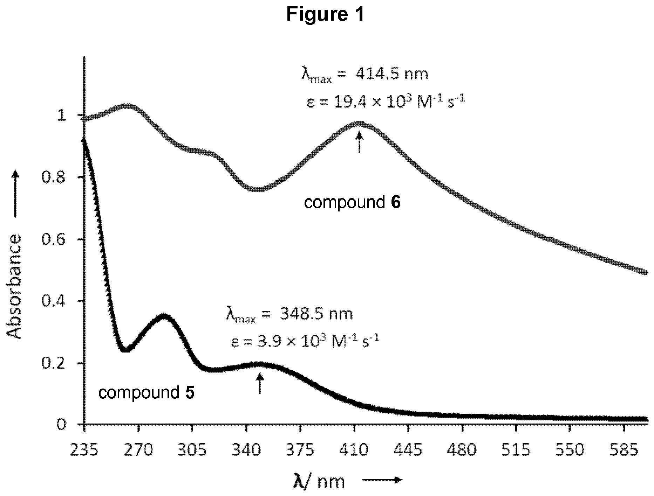

- **Figure 1:**: UV-Vis absorption spectra of TMP-PC-Cl (black) and 4-CmTMP-PC-Cl (red). A solution of 50 µM of the respective dimerizer in PBS buffer (pH 7.4, 0.5% DMSO) was subjected to UV-Vis absorption analysis (Figure 1). 0.5%DMSO in PBS was used as the blank. TMP-PC-Cl shows an absorption peak at 348.5 nm, which indicates the presence of the 2-nitrobenzyl photo-cleavable moiety. 4-CmTMP-PC-Cl shows an additional absorption peak at 414.5 nm, which is attributed to the presence of the photo-caging diethylaminocoumarinyl group.

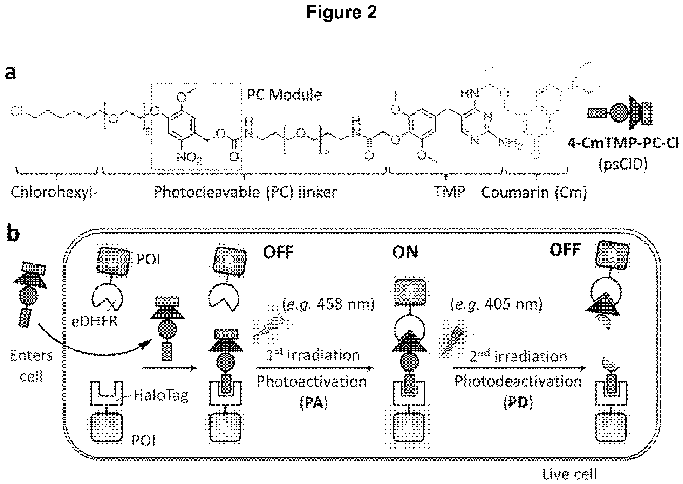

- **Figure 2:**: a) Chemical structure of the psCID, 4-CmTMP-PC-Cl, featuring a chlorohexyl- ligand for covalent binding with HaloTag, a linker containing a photocleavable (PC) 2-nitrobenzyl module, and a diethylaminocoumarinyl-caged TMP ligand.
b) Schematic view of 4-CmTMP-PC-Cl for reversibly control of protein dimerization in a live cell using light. 4-CmTMP-PC-Cl is readily cell permeable and first pre-localized to the protein of interest (POI) A fused with HaloTag. Afterwards, a first light illumination decages the diethylaminocoumarinyl group and exposes TMP ligand to binding with eDHFR-fused POI B. This step is called photoactivation (**PA**). Upon a second light illumination (e.g. using a different wavelength of light), the photocleavable linker is cleaved, leading to the dissociation of the dimerization process. This step is called photodeactivation (**PD**).

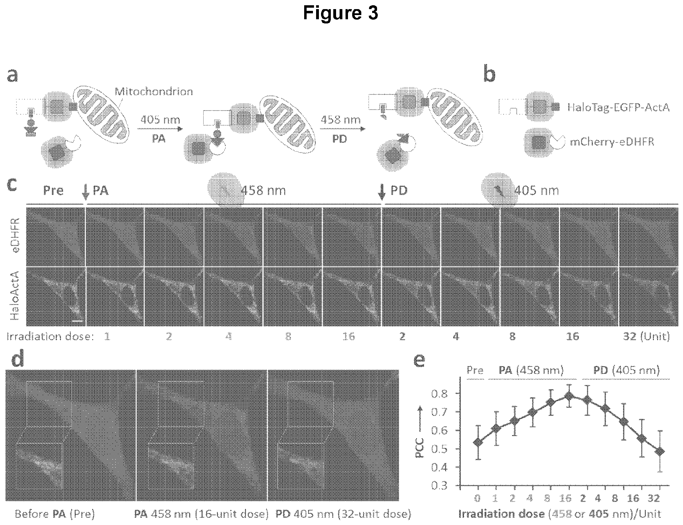

- **Figure 3:**: Reversible targeting to mitochondria using 4-CmTMP-PC-Cl under the control of orthogonal illumination wavelengths.
a) Schematic view of the assay.
b) The two constructs used in the assay.
c)HaloTag-EGFP-ActA (lower panel) is localized at mitochondria while mCherry-eDHFR (upper panel) is largely cytosolic before photoactivation (**PA**); herein, the C-terminal ActA peptide sequence (LILAMLAIGVFSLGAFIKIIQLRKNN) is responsive for targeting the protein to mitochondria; the whole cell was irradiated with increasing doses of 458 nm light, which gradually induced the targeting of mCherry-eDHFR from cytosol to mitochondria; afterwards, the dimerizer was photo-deactivated (**PD**) by applying increasing doses of 405 nm laser irradiation, which gradually reversed the mitochondria localization of mCherry-eDHFR.
d) Enlarged images of three key frames in c).
e) Pearson's correlation coefficient (PCC) analysis of the colocalization between mCherry and EGFP (n = 10 cells).

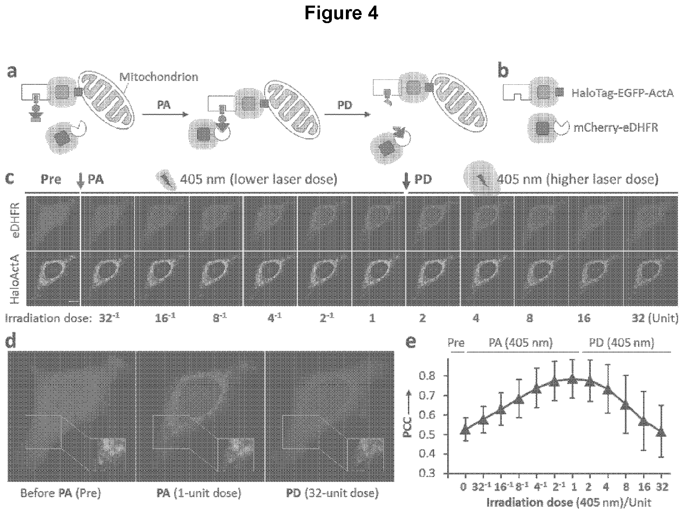

- **Figure 4:**: Photoactivation and photodeactivation of the 4-CmTMP-PC-Cl using a single wavelength of light (405 nm).
a)Schematic view of the assay.
b)The two constructs used in the assay.
c)HeLa cells co-expressing mCherry-eDHFR and HaloTag-EGFP-ActA were illuminated with increasing doses of 405 nm light which gradually targeted mCherry-eDHFR from cytosol to mitochondria; after the irradiation dose exceeds one unit, higher density of 405 nm irradiation started to induce the dedimerization that relocates mCherry-eDHFR from mitochondria to cytosol.
d)Enlarged images of three key frames in c).
e)Pearson's correlation coefficient (PCC) analysis of the colocalization between mCherry and EGFP.

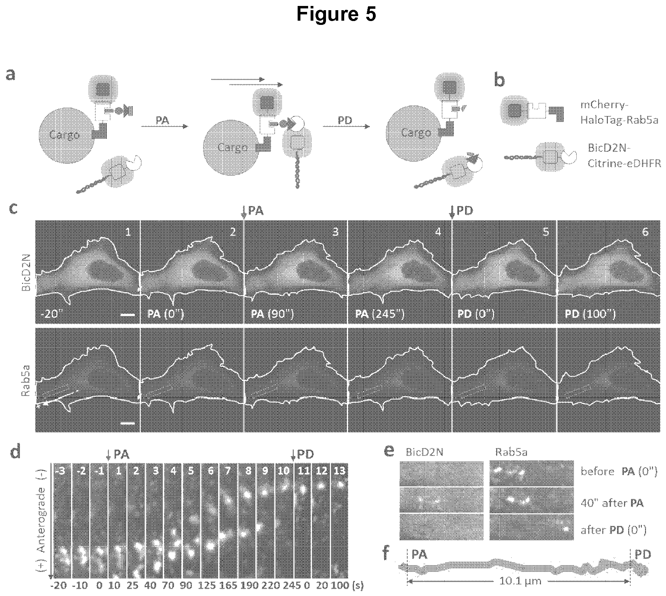

- **Figure 5:**: Spatiotemporal control of intracellular dynein-cargo motility by light using 4-CmTMP-PC-Cl. The dynein binding domain of the dynein adaptor protein Bicaudal D2 (1-594), i.e. BicD2N, was reversibly targeted to Rab5a-localized early endosomes (EEs). Recruitment of BicD2N to EE recruits and activate cytoplasmic dynein and stimulate processive motility of EEs.
a)Schematic view of the assay. Photoactivation allows the recruitment of BicD2N from cytosol to EEs to activate dynein. Subsequently, EEs start to migrate toward microtube organization center (MTOC) in the central part of the cell. After photodeactivation, BicD2N dissociates from EEs, leading to immediate disruption of cargo transport.
b)The two constructs used in the assay.
c)HeLa cells co-expressing BicD2N-Citrine-eDHFR and mCherry-HaloTag-Rab5a were treated with 4-CmTMP-PC-Cl (1.0-1.2 µM) and then washed before imaging. BicD2N was largely cytosolic (upper panels) while mCherry-HaloTag-Rab5a was located at EEs (lower panels) before **PA** (#1, 2). Upon **PA,** BicD2N-Citrine-eDHFR was recruited to EEs to initiate migration of Rab5a-labeled EEs to MTOC (#3, 4). Upon **PD,** BicD2N-Citrine-eDHFR rapidly dissociates from the cargo and diffuses to the cytosol within seconds, whereas EEs remain largely localized near MTOC (#5, 6).
d)Analysis of the migration of a single EE: A single EE (indicated by the red triangle, located at the blue frame region in **c**) was almost steady before **PA** and started to migrate upon **PA**; its migration was disrupted upon **PD.**
e)BicD2N was recruited to individual EEs after **PA** and dissociated from EEs after **PD.**
f) The trajectory of the individual EE in **d)** before **PA,** after **PA** and after **PD** (10" interval between each dot).
- **Figure 6:**: Statistical analysis of EE migration rate before **PA,** after **PA** and after **PD** (n = 13 vesicles).
- **Figure 7:**: UPLC chromatogram of **compound 6** with a retention time of 2.65 min.

### Examples

### Example 1:

### Synthesis of compound 6:

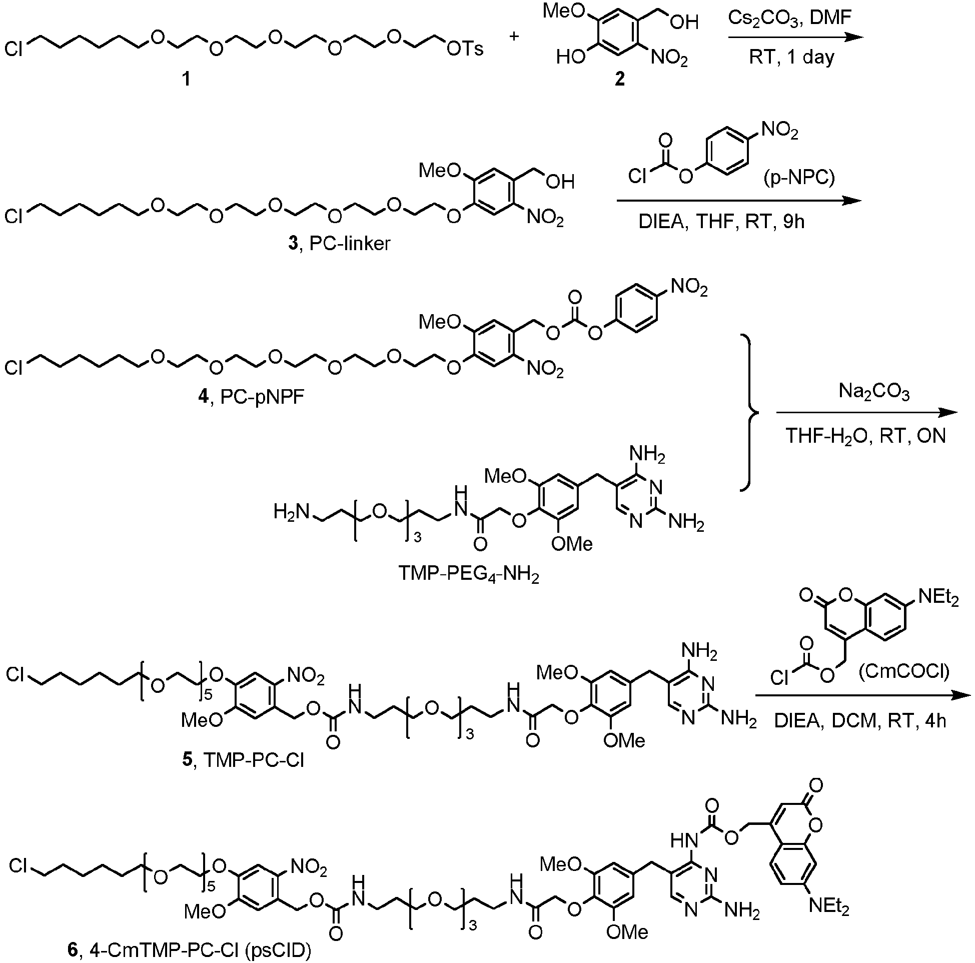

**Abbreviations:** DMF: dimethylformamide, RT: room temperature, PC-linker: photocleavable linker, p-NPC: p-nitrophenyl chloroformate, DIEA: *N,N-*diisopropylethylamine, THF: tetrahydrofuran, ON: overnight, PEG: polyethylene glycol, TMP: trimethoprim, DCM: dichloromethane, Cm: diethylaminocoumarinyl group.

### Compound 3: (4-((21-Chloro-3,6,9,12,15-pentaoxahenicosyl)oxy)-5-methoxy-2-nitrophenyl)methanol.

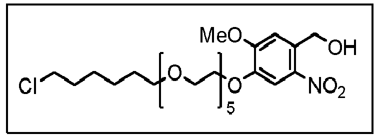

4-(Hydroxymethyl)-2-methoxy-5-nitrophenol (250 mg, 0.906 mmol, from Chemspace # BBV-33813491) and Cs₂CO₃ (443 mg, 1.36 mmol) were combined in a two-necked roundbottom flask (RBF) and anhydrous DMF (3 ml) was injected under a flush of Ar. CI-PEO-OTs (600 mg, 1.18 mmol) was injected dropwise and the reaction solution was stirred under Ar at room temperature (RT) overnight (ON). After workup, the reaction mixture was purified by silica gel chromatography using EtOAc as the eluent. Approximately 463 mg yellowish viscous oil was obtained in a yield of ∼ 95%. **¹H-NMR** (CDCl₃, 400MHz): *δ* 7.78 (s, 1 H), 7.17 (s, 1 H), 4.96 (s, 2H), 4.26 (t, *J*=4.88Hz, 2H), 3.98 (s, 3H), 3.91 (t, *J*=4.6Hz, 2H), 3.73 (m, 2H), 3.7-3.6 (m, 12H), 3.57 (m, 2H), 3.53 (t, *J*=6.72Hz, 2H), 3.45 (t, *J*=6.64Hz, 2H), 1.77 (p, *J*=7.88Hz, 2H), 1.59 (p, *J*=7.56Hz, 2H), 1.45 (m, 2H), 1.36 (m, 2H); **¹³C-NMR** (CDCl₃, 400MHz): *δ* 154.42, 147.33, 139.66, 132.57, 111.29, 110.27, 71.26, 70.97, 70.65, 70.62, 70.60, 70.12, 69.53, 69.11, 62.84, 56.41, 45.05, 32.56, 29.47, 26.71, 25.44; **HRMS**(ESI): C₂₄H₄₁ClNO₁₀⁺ calcd. 538.2414, found 538.2426 [M+H]⁺.

### Compound 4: 4-((21-Chloro-3,6,9,12,15-pentaoxahenicosyl)oxy)-5-methoxy-2-nitrobenzyl (4-nitrophenyl) carbonate

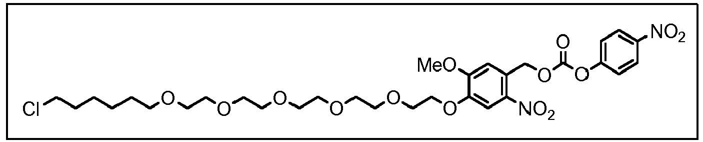

PC-linker (463 mg, 0.86 mmol) was dissolved in anhydrous THF (2.9 ml) under a flush of Ar. *p*-Nitrophenol chloroformate (208 mg, 1.03 mmol) and diisopropylethylamine (DIEA, 166 mg, 1.29 mmol) were added and the resultant reaction solution was stirred at RT for 6h. Additional *p*-nitrophenol chloroformate (208 mg, 103 mmol) and DIEA (166 mg, 1.29 mmol) were added and the reaction solution was further stirred for 3h to allow complete conversion of PC-linker intermediate. After workup, the reaction mixture was purified via silica gel chromatography to afford 538 mg light yellow oil as the amine-reactive chloroformate intermediate **4. LC-MS** (C18, 254 nm, MeCN/H₂O): *t*_{R} 7.11 min, *m*/*z* 702.6 [M+H]⁺ (C₃₁H₄₄ClN₂O₁₄⁺, calcd. 703.2476). **¹H-NMR** (CDCl₃, 400MHz): *δ* 8.30 (d, J=9.12Hz, 2H), 7.84 (s, 1 H), 7.41 (d, J=9.12Hz, 2H), 7.09 (s, 1 H), 5.70 (s, 2H), 4.27 (t, J=4.92Hz, 2H), 3.99 (s, 3H), 3.92 (t, J=4.52Hz, 2H), 3.73 (m, 2H), 3.68 (m, 2H), 3.64-66 (m, 8H), 3.63 (m, 2H), 3.57 (m, 2H), 3.52 (t, J=6.68Hz, 2H), 3.45 (t, J=6.64Hz, 2H), 1.77 (p, J=7.64Hz, 2H), 1.59 (p, J=7.36Hz, 2H), 1.44 (m, 2H), 1.37 (m, 2H); **¹³C-NMR** (CDCl₃, 400MHz): *δ* 155.35, 154.09, 152.06, 148.16, 145.53, 139.83, 125.38, 125.24, 121.73, 110.86, 110.24, 77.20, 71.23, 70.93, 70.63, 70.57, 70.08, 69.42, 69.11, 67.72, 56.50, 45.04, 32.53, 29.43, 26.68, 25.40; **DEPT135** (CDCl₃, 400MHz): *δ*(+) 71.23, 70.93, 70.63, 70.60, 70.57, 70.08, 69.42, 69.11, 67.72, 56.51, 45.04, 32.52, 29.43, 26.68, 25.40; *δ*(-)125.38, 121.73, 110.87, 110.24, 56.51; **HRMS** (ESI): C₃₁H₄₄O₁₄N₂Cl⁺, calcd. 703.2476, found 703.2486 [M+H]⁺.

### Compound 5: 4-((21-Chloro-3,6,9,12,15-pentaoxahenicosyl)oxy)-5-methoxy-2-nitrobenzyl (1-(4-((2,4-diaminopyrimidin-5-yl)methyl)-2,6-dimethoxyphenoxy)-2-oxo-7,10,13-trioxa-3-azahexadecan-16-yl)carbamate

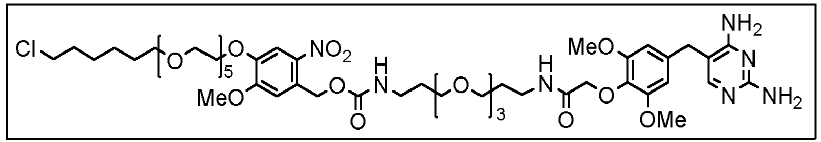

In a typical reaction, TMP-PEG₃-NH₂.nTFA (61.4 mg, 0.114 mmol, 1.3 eq.) was dissolved in sat. Na₂CO₃ (1.44 ml) in a RBF equipped with a stir bar. A solution of Pc2-pNPF (61.8 mg, 0.088 mmol, 1.0 eq.) in THF (0.44 ml) was injected dropwise to the stirring solution under Ar. The reaction mixture was stirred at RT for 4h. After workup, the reaction mixture was purified by silica gel chromatography to afford 25.6 mg yellowish solid as the product in a yield of 26 %. **¹H-NMR** (CDCl₃, 500MHz): *δ* 7.81 (t, *J*=5.10Hz, 1H, -CON*H*-), 7.72 (s, 1H), 7.63 (s, 1H), 6.99 (s, 1H), 6.37 (s, 2H), 5.86 (t, *J*=5.45Hz, 1H, -OCON*H*-), 5.44 (s, 2H, ArC*H*₂OCONH-), 5.31 (s, br, 2H, -N*H*₂), 5.10 (s, br, 2H, -N*H*₂), 4.45 (s, 2H, ArOC*H*₂CONH-), 4.21 (t, *J*=4.7Hz, 2H, ArOC*H*₂CH₂O-), 3.91 (s, 2H, Ar-C*H*₂-Ar')), 3.88 (t, *J*=4.6Hz, 2H, ArOCH₂C*H*₂O-), 3.80 (s, 3H, -OC*H*₃), 3.79 (s, 6H, -(OC*H*₃)₂), 3.71 (t, *J*=4.75Hz, 2H, - C*H*₂Cl), 3.67-3.48 (m, 28H), 3.43 (t, *J*=6.65Hz, 2H), 3.39 (m, 2H), 3.30 (m, 2H), 1.81 (m, 2H), 1.75 (m, 4H), 1.57 (m, 2H), 1.42 (m, 2H), 1.34 (m, 2H); **¹³C-NMR** (CDCl₃, 500MHz): *δ* 169.87, 162.87, 160.82, 155.99,153.90, 153.90, 153.58, 152.51, 147.20, 139.43, 136.77, 135.38, 134.12, 133.27, 128.73, 126.09, 115.69, 110.32, 109.89, 106.39, 104.93, 104.88, 72.58, 71.17, 70.78, 70.50, 70.47, 70.41, 70.19, 70.03, 69.99, 69.38, 69.33, 68.89, 63.25, 60.83, 56.27, 56.09, 56.04, 45.04, 39.14, 36.39, 34.37, 32.47, 29.64, 29.36, 29.32, 26.63, 25.34; **DEPT135** (CDCl₃, 500MHz): (+) *δ* 72.63, 71.22, 70.83, 70.55, 70.52, 70.24, 70.87, 70.05, 69.44, 69.39, 68.94, 63.31, 45.09, 39.20, 36.45, 34.42, 32.52, 29.42, 29.38, 26.68, 25.40; (-) *δ* 153.48, 126.14, 115.75, 110.37, 109.94, 104.99, 104.94, 60.88, 56.33, 56.14, 56.09; **HRMS (ESI):** C₅₀H₇₉ClN₇O₁₈⁺ [M+H]⁺, calcd. 1100.5165, found 5129.

### Compound 6: 4-((21-chloro-3,6,9,12,15-pentaoxahenicosyl)oxy)-5-methoxy-2-nitrobenzyl (1-(4-((2-amino-4-((((7-(diethylamino)-2-oxo-2H-chromen-4-yl)methoxy)carbonyl)amino)pyrimidin-5-yl)methyl)-2,6-dimethoxyphenoxy)-2-oxo-7,10,13-trioxa-3-azahexadecan-16-yl)carbamate

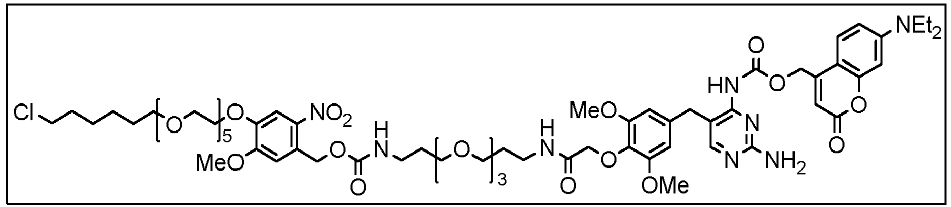

In a typical reaction, TMP-Pc2-Cl (38.1 mg, 0.0346 mmol), and CoumCOCI (54.1 mg, 0.175 mmol) were combined in a dried small RBF equipped with a stir bar. Anhydrous DCM (0.876 ml) was injected and DIEA (116 µl, 87.6 mg, 0.677 mmol) was added dropwise. The clear reaction solution was stirred at RT for 10 h. After workup, the reaction mixture residue was first purified by silica gel chromatography and then by preparative HPLC-MS (C18 column, ∅ 21 mm) to give 6.2 mg **compound 6** as the target molecule (*t*_{R} 7.9 min) in a yield of 13 %. **HRMS**(ESI): C₆₅H₉₄O₂₂N₈Cl⁺, calcd. 1373.6166, found 1373.6176 [M+H]⁺. **U-HPLC/MS**(ESI)**:** *t*_{R} 2.65min, >99% purity.

### Example 2:

UV-Vis absorption spectra of TMP-PC-Cl and 4-CmTMP-PC-Cl.

UV-Vis absorption spectra were recorded using SHIMADZU UV-2401PC UV-Vis Recording Spectrophotometer. A solution of 50 µM of the respective dimerizer in PBS buffer (pH 7.4, 0.5% DMSO) was subjected to UV-Vis absorption analysis (**Figure 1**). 0.5% DMSO in PBS was used as the blank. TMP-PC-Cl shows an absorption peak at 348.5 nm, which indicates the presence of the 2-nitrobenzyl photo-cleavable moiety. 4-CmTMP-PC-Cl shows an additional absorption peak at 414.5 nm, which is attributed to the presence of the photo-caging diethylaminocoumarinyl group.

### Example 3:

Reversible targeting to mitochondria using 4-CmTMP-PC-Cl was controlled by orthogonal illumination wavelengths (**Figure 3**). Imaging was carried out ∼24h post transfection of HeLa cells using a 4-well or 8-well imaging chamber (SARSTEDT x-well slide) in Dulbecco's Modified Eagle Medium (gibco life technologies, REF: 21063-29) supplemented with additional 10% FBS, 1% sodium pyruvate, 1% NEAA and 1% penicillin-streptomycin at 37°C under 5% CO₂, using the Zeiss LSM 510 inverted scanning confocal microscope (EC Plan-NEOFLUAR, 40 x, oil, NA 1.3 objective) equipped with a 405 nm laser. HeLa cells were treated with psCID dimerizer at the concentration of 1.0-1.2 µM for 30min and then washed twice by PBS before imaging. The photoactivation step should be applied within 20-30min after washing to achieve optimal result. In this patent, one unit of 405nm light illumination dose is defined as 0.8 µs/pixel irradiation at 80% power (150µW) using a 405 nm laser diode, while one unit of 458 nm light illumination dose is defined as 6.4 µs/pixel irradiation with 50% output at 100% power applying the 458 nm Ar laser line, Both light illuminations are performed in a 512 pixel × 512 pixel image (56.3 µm x 56.3 µm). Light doses higher than one unit was achieved by proportionally increase the irradiation time while light dose less than one unit was achieved by proportionally reduce the laser power. Photoactivation was performed by applying increasing doses of 458 nm light irradiation while photodeactivation was achieved by applying increasing doses of 405 nm light within a defined region of photoactivation (ROP).

### Example 4:

Light-induced targeting to mitochondria can be tuned by application of different does of light illumination at 405 nm (**Figure 4**). Photoactivation was performed by applying increasing dose (1/32 to 1 unit) of 405 nm light irradiation while photodeactivation was achieved by applying increasing doses (1 to 32 units) of 405 nm irradiation within a defined region of photoactivation (ROP).

### Example 5:

Reversible control of cytoplasmic dynein motor protein function for early endosome (EE) transport using the psCID system (**Figure 5**). In this example, the motor binding domain of the dynein adaptor protein Bicaudal D2 (1-594), i.e. BicD2N, was reversibly targeted to Rab5a-localized early endosomes (EEs) using light. Recruitment of BicD2N to EE will recruit and activate cytoplasmic dynein and stimulate processive motility of EEs. Imaging, cell seeding, transfection, microscopy, photoactivation and photodeactivation were performed using the procedures described for **Figure 4****.**

### Example 6:

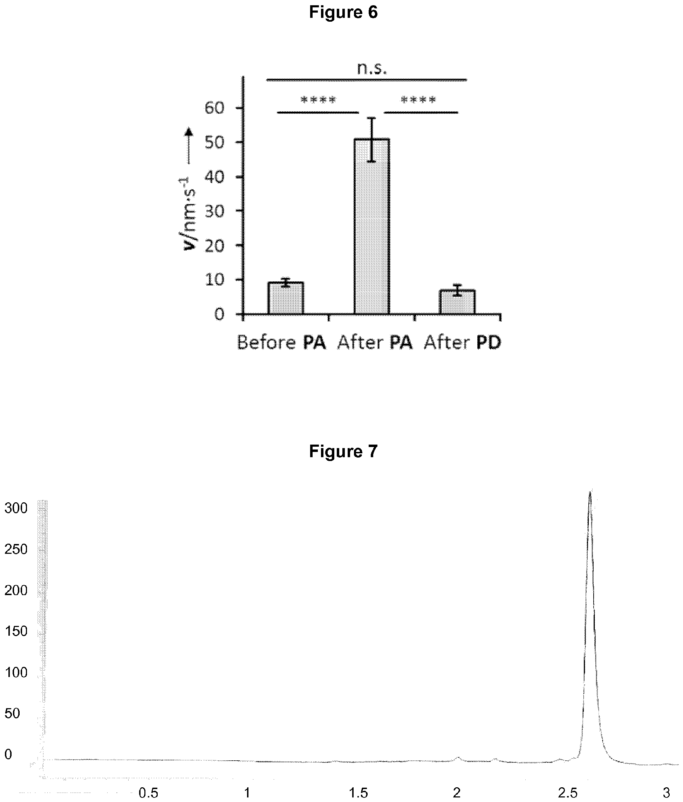

Statistical analysis of EE migration rate before PA, after PA and after PD (n = 13 vesicles) (**Figure 6**). Before PA and after PD, EEs show non-directional migration with an average migration speed of < 10 nm/s. After PA, EEs show an enhanced and directional migration speed of > 50 nm/s. Student's t-text reveals that migration speed change is significant after PA while the change of migration speed before PA and after PD is non-significant (n.s). The vesicles for chosen in this analysis are fully tractable during the migration process and the migration length is no less than 10 µm.

## Claims

1. A compound of general formula (I)
Hal-(CH₂)₆-**F¹-P-F²-E** (**I**)
wherein
Hal is selected from -Cl, -Br and -I;
**E** is selected from: 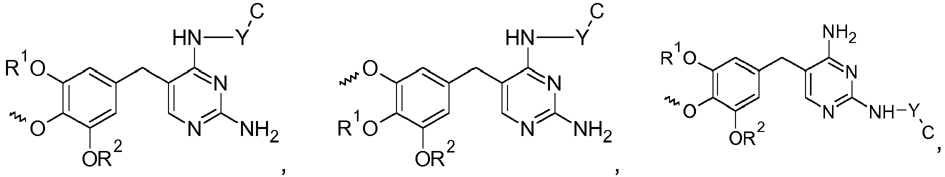 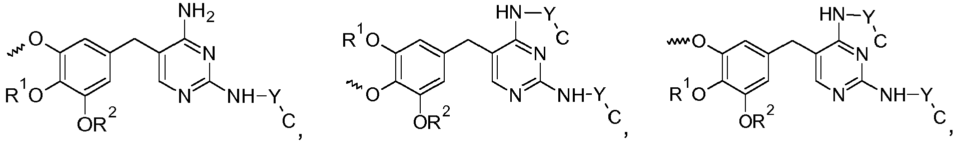 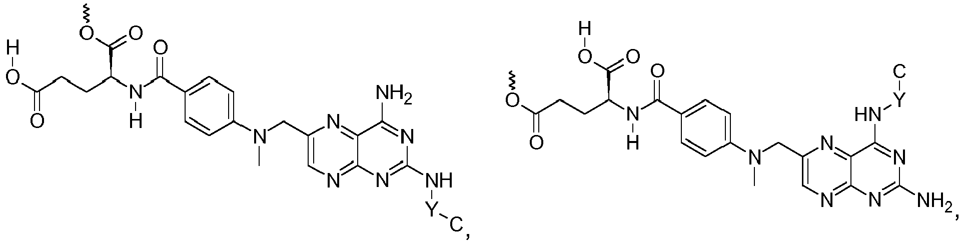 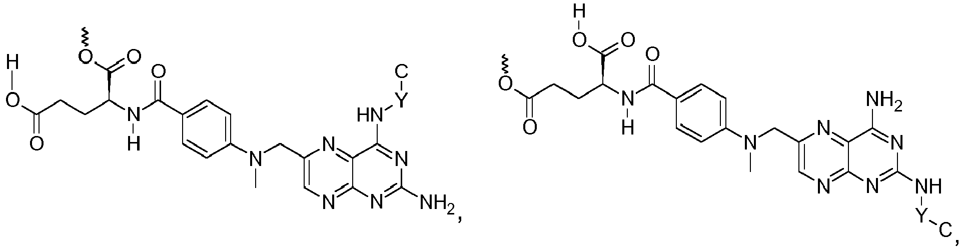 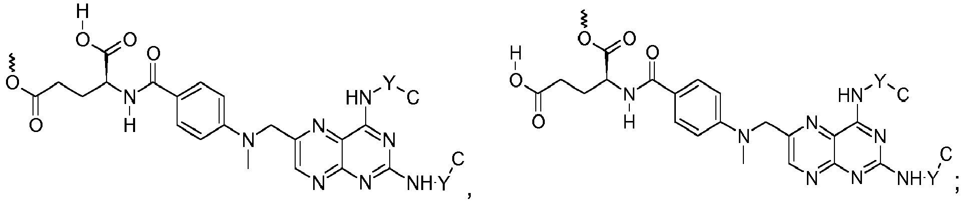
wherein R¹ and R² are independently of each other selected from: -H, -CH₃, -C₂H₅, -C₃H₇, -Ph, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, cyclo-C₃H₅, cyclo-C₄H₇, cyclo-C₅H₉, cyclo-C₆H₁₁;
Y represents a bond, -CH₂-, -NHR³⁴-, -O-, -S-, -C(=O)-O-, -O-C(=O)-, -CO-, -NHC(=O)-, -C(=O)NH-, -NR³⁴-C(=O)-, -C(=O)NR³⁴-, -NH-C(=S)-, or -C(=S)NH-;
C is selected from -C1, -C2, -C3, -C4, -C5, -C6, -C7, -C8, -C9, and -C10;
| | | | |
|---|---|---|---|
| C1 is | 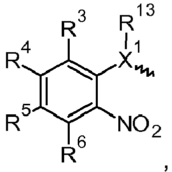 | C2 is | 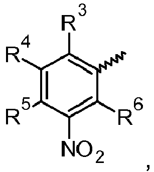 |
| C3 is | 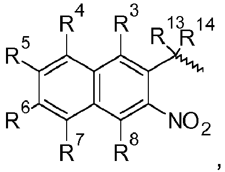 | C4 is | 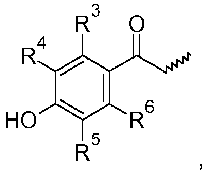 |
| C5 is | 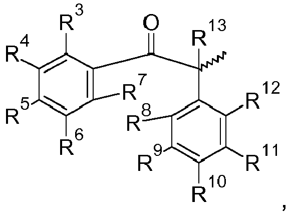 | C6 is | 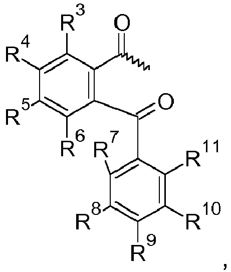 |
| C7 is | 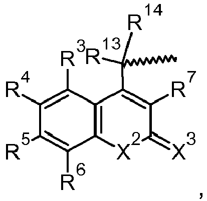 | C8 is | 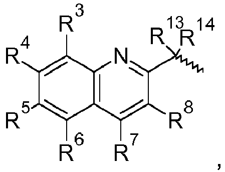 |
| C9 is | 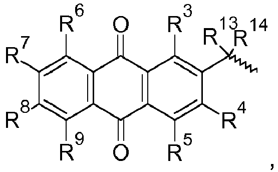 | C10 is | 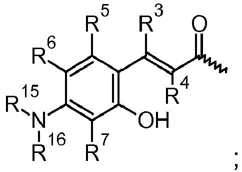 |
P is selected from -P1-, -P2-, -P3-, -P4-, -P5-, -P6-, -P7-, -P8-, -P9-, -P10-, -P11-, and -P12-; wherein
| | | | |
|---|---|---|---|
| P1 is | 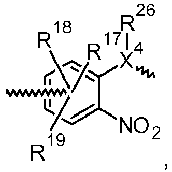 | P2 is | 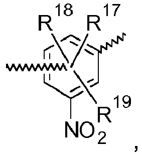 |
| P3 is | 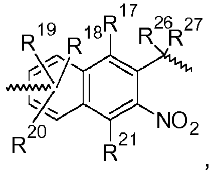 | P4 is | 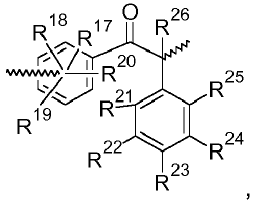 |
| P5 is | 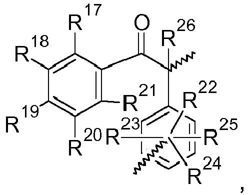 | P6 is | 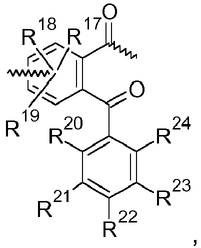 |
| P7 is | 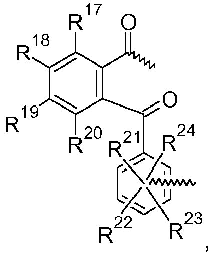 | P8 is | 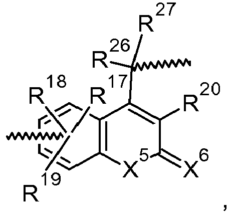 |
| P9 is | 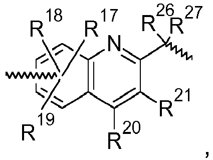 | P10 is | 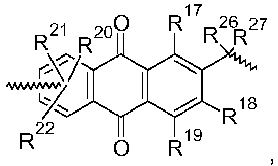 |
| P11 is | 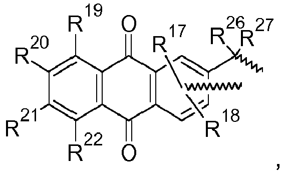 | P12 is | 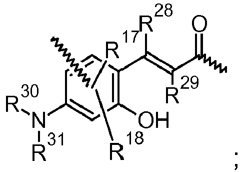 |
wherein if C is C1, P cannot be P1 or P2;
wherein if C is C2, P cannot be P1 or P2;
wherein if C is C3, P cannot be P3;
wherein if C is C4, P cannot be P4 or P5;
wherein if C is C5, P cannot be P4 or P5;
wherein if C is C6, P cannot be P6 or P7;
wherein if C is C7, P cannot be P8;
wherein if C is C8, P cannot be P9;
wherein if C is C9, P cannot be P10 or P11;
wherein if C is C10, P cannot be P12;
X¹ is either 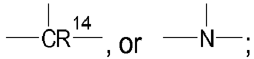
X⁴ is either 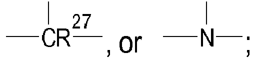
X² and X⁵ are independently of each other selected from: -O-, -S-, -NH-, and -NR³²₋;
X³ and X⁶ are independently of each other selected from: =O, =S, =NH, and =NR³³;
F¹ is -A¹-L^{A}-B¹- and F² is -A²-L^{B}-B²-, wherein
A¹, A², B¹ and B² represent independently of each other -CH₂-, -NH-, -O-, -S-, -CO-, -NH-CO-, -CO-NH-, -NH-CO-NH-, -O-CO-, -O-CO-O-, -NH-CO-O-, -O-CO-NH-, -NH-CO-CH₂-, -CH₂-CO-NH- and -CO-O-;
L^{A} and L^{B} represent independently of each other -(CH₂)ₘ₁-, -(CH₂)ₘ₂-, -(CH₂)ₘ₁-CHR³⁵-(CH₂)ₘ₂-, -(CH₂)ₘ₁-CR³⁶R³⁷-(CH₂)ₘ₂-, -(C₂H₄O)ₘ₁-, -(C₂H₄O)ₘ₂-, -(OC₂H₄)ₘ₁-, -(OC₂H₄)ₘ₂-, -(CH₂)ₘ₅-(C₂H₄O)ₘ₆-, -(CH₂)ₘ₅-(OC₂H₄)ₘ₆-, -(C₂H₄O)ₘ₅-(CH₂)ₘ₆-, -(OC₂H₄)ₘ₅-(CH₂)ₘ₆-, -(CH₂)ₘ₇-(C₂H₄O)ₘ₈-, —(CH₂)ₘ₇-(OC₂H₄)ₘ₈-, -(C₂H₄O)ₘ₇-(CH₂)ₘ₈-, -(OC₂H₄)ₘ₇-(CH₂)ₘ₈-, -(CH₂)ₘ₁-(C₂H₄O)ₘ₂-(CH₂)ₘ₅-, -(CH₂)ₘ₁-(OC₂H₄)ₘ₂-(CH₂)ₘ₅-, -(CH₂)ₘ₆-(C₂H₄O)ₘ₇(CH₂)ₘ₈-, -(CH₂)ₘ₆-(OC₂H₄)ₘ₇-(CH₂)ₘ₈-, -o-C₆H₄-, -m-C₆H₄-, -p-C₆H₄-, 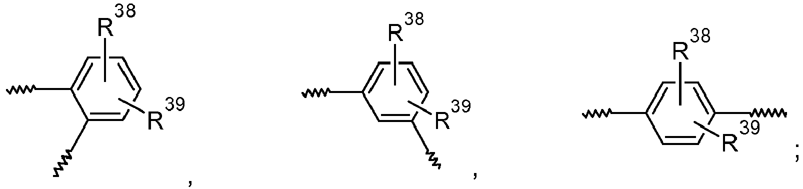
R³ to R¹⁴ , R¹⁷ to R²⁹ and R³⁵ to R³⁹ represent independently of each other -H, -F, -Cl, -Br, -I, -CF₃, -NH₂, -N(CH₃)₂, -N(C₂H₅)₂, 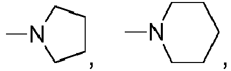 -OH, -OCH₃, -OC₂H₅, -OC₃H₇, -OCH₂COOH, -N(CH₂COOH)₂, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH₂NH₂, -CH₂OH, -CH₂SH, -CH₂-CH₂NH₂, -CH₂-CH₂SH, -C₆H₄-OCH₃, -C₆H₄-OH, -CH₂-CH₂-OCH₃, -CH₂-CH₂OH, -CH₂-OCH₃, -CH₂-C₆H₄-OCH₃, -CH₂-C₆H₄-OH, or
two neighbouring residues R³ to R¹² and R¹⁷ to R²⁵ form a benzo ring, or
three neighbouring residues R³ to R¹² and R¹⁷ to R²⁵ form a 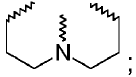
R¹⁵, R¹⁶ , R³⁰ to R³⁴ represent independently of each other -H, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH₂NH₂, -CH₂OH, -CH₂SH, -CH₂-CH₂NH₂, -CH₂-CH₂SH, -C₆H₄-OCH₃, -C₆H₄-OH, -CH₂-CH₂-OCH₃, -CH₂-CH₂OH, -CH₂-OCH₃, -CH₂-C₆H₄-OCH₃, -CH₂-C₆H₄-OH,
m1, m2, m5, m6, m7 and m8 represent independently of each other an integer from 1 to 20;
m3 and m4 represent independently of each other an integer from 0 to 5.

2. The compound according to claim 1 of general formula (**I-A**) 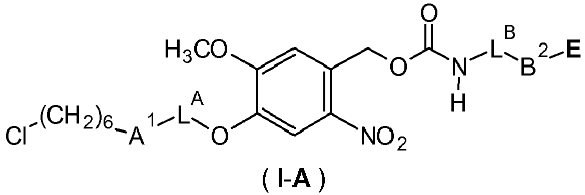 wherein
**E** is selected from: 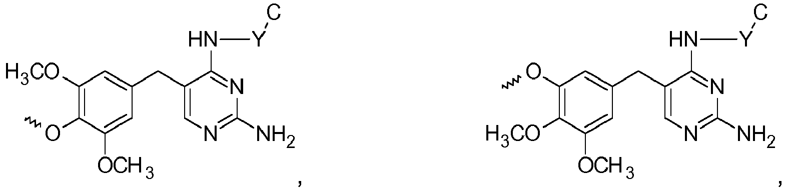 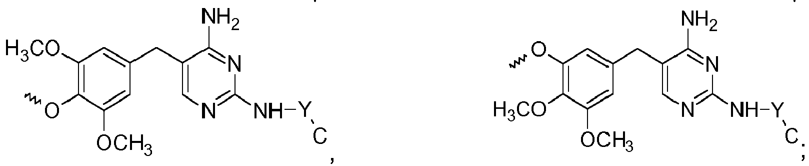 Y-C is 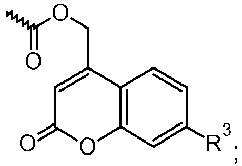 R³ is selected from -NH₂, -N(CH₃)₂, -N(C₂H₅)₂, 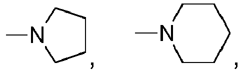 and -N(CH₂COOH)₂; and
wherein A¹, L^{A}, L^{B} and B² have the meanings as defined in claim 1.

3. The compound according to claim 1 of general formula (**I-B**) 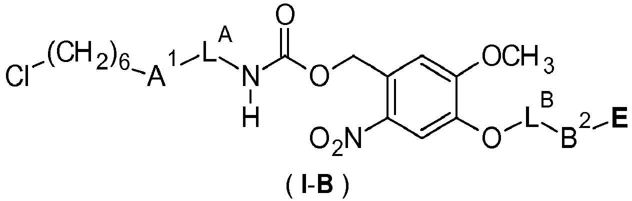 wherein
**E** is selected from: 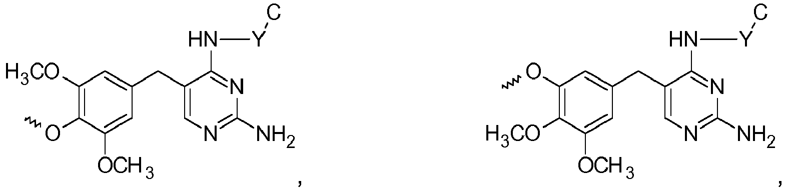 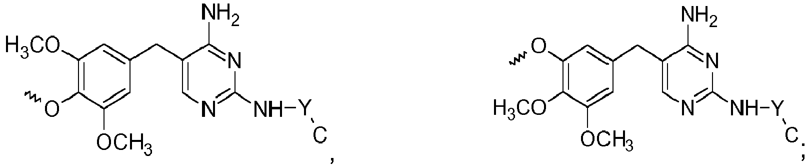 Y-C is 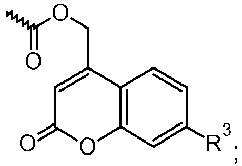 R³ is selected from -NH₂, -N(CH₃)₂, -N(C₂H₅)₂, 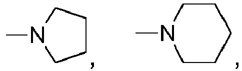 and -N(CH₂COOH)₂; and
wherein A¹, L^{A}, L^{B} and B² have the meanings as defined in claim 1.

4. Chemo-optogenetic system for testing intracellular protein interaction in cells, comprising:
a) the compound according to claim 1;
b) fusion protein 1 comprising a test compound 1 and at least HaloTag; and
c) fusion protein 2 comprising a test compound 2 and at least the TMP binding domain of a bacterial DHFR.

5. Chemo-optogenetic system according to claim 4, wherein fusion protein 1 and/or fusion protein 2 comprise further a component for identification and/or purification of the fusion proteins and/or a targeting peptide or protein.

6. Chemo-optogenetic system according to claim 4 or 5, wherein test compound 1 and test compound 2 are selected independently of each other among gene products, proteins, protein domains, peptides, polypeptides, glycopeptides, proteins with secondarily modified amino acids, peptides or proteins with protecting groups, saccharides, small molecules, lipids, polynucleotides, oligonucleic acids, DNA and RNA.

7. Chemo-optogenetic system according to any of claims 4 to 6, wherein the bacterial DHFR is eDHFR.

8. Chemo-optogenetic system according to any of claims 4 to 7, wherein the compound has the structure of formula (**I-A**) or (**I-B**).

9. Use of the chemo-optogenetic system as defined in any of claims 4 to 8 for testing the interactions of a test compound 1 with a test compound 2.

10. Method for testing intracellular protein interaction in cells, comprising the following steps:
a) Providing, transfecting and expressing the DNA sequence of a fusion protein 1 comprising a test compound 1 and at least HaloTag;
b) providing, transfecting and expressing the DNA sequence of a fusion protein 2 comprising a test compound 2 and at least the TMP binding domain of a bacterial DHFR;
c) adding compound according to claim 1 to cells and letting them pass the plasma membrane;
d) activating and/or deactivating the compound according to claim 1 with light under irradiation condition A for activation and under irradiation condition B for deactivation; and
e) determining the change in a selected test parameter system.

11. Method according to claim 10, wherein the irradiation condition A corresponds to irradiation with an Argon laser at a wavelength of 458 nm and the irradiation condition B corresponds to irradiation with a laser diode at a wavelength of 405 nm.

12. Method according to claim 10, wherein the irradiation condition A and the irradiation condition B correspond to irradiation with a laser diode at a wavelength of 405 nm and wherein the applied fluence of the laser under the irradiation condition A is lower than about 0.99 J/cm² and the applied fluence of the laser under the irradiation condition B is higher than about 0.99 J/cm².

13. Method according to any of claims 10 to 12, wherein test compound 1 and test compound 2 are selected independently from one another among gene products, proteins, protein domains, peptides, polypeptides, glycopeptides, proteins with secondarily modified amino acids, peptides or proteins with protecting groups, saccharides, small molecules, lipids, polynucleotides, oligonucleic acids, DNA and RNA.

14. Intermediate compound of the general formula **(I-1-A)** or **(I-1-B),** 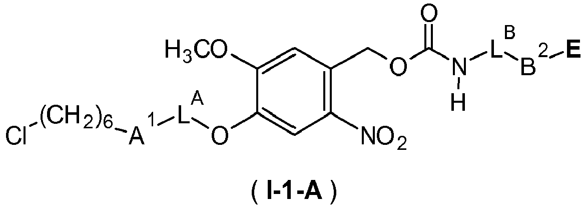 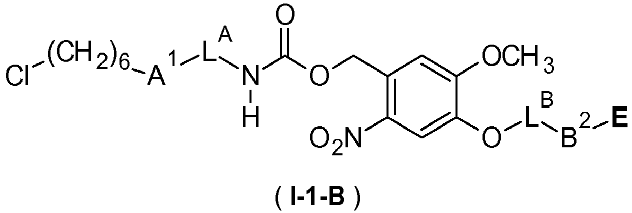 wherein
the moieties **A¹, B², L^{A}, L^{B}, and E** have the meanings as defined in claim 2 and
wherein the residue -YC present in the moiety **E** represents hydrogen (-H).

15. Kit, comprising
a) the compound according to claim 1,
b) the nucleotide sequences or the vectors including the nucleotide sequences coding for at least HaloTag and respectively a bacterial DHFR.
